# EUROPEAN PATENT SPECIFICATION

(11) **EP 4 419 158 B1**
(45) Date of publication and mention of the grant of the patent: **26.11.2025**
(21) Application number: 22803439.3
(22) Date of filing: 21.10.2022
(51) Int. Cl.: A61M 5/19, A61M 5/31, A61M 5/315, A61M 5/32, A61N 5/10

(54) **SYSTEMS FOR PRODUCING MIXTURES**
SYSTEME ZUR HERSTELLUNG VON MISCHUNGEN
SYSTÈMES DE PRODUCTION DE MÉLANGES

(30) Priority: 22.10.2021 US 202163270906 P
(43) Date of publication of application: 28.08.2024
(73) Proprietor: Boston Scientific Medical Device Limited, Galway (IE); Boston Scientific Scimed, Inc., Maple Grove, MN 55311-1566 (US)
(72) Inventor: CLEVELAND, Benjamin, Bellingham, Massachusetts 02019 (US); BAVISKAR, Nitesh Ghananil, Maharashtra, Kalyan West 421301 (IN); SHAIKH, Junaid Mohammed, Limbayat, Gujarat, Surat 395002 (IN); GRAFFAM, Richard Earl, Pelham, New Hampshire 03076 (US); HERNANDEZ, Joseph, Rutland, Massachusetts 01543 (US); WATSON, Christopher, Lincoln, Massachusetts 01773 (US); CREEGAN, Jennie, Minneapolis, Minnesota 55419 (US); KOLSTE, Kolbein, Acton, Massachusetts 01720 (US)
(74) Representative: Pfenning, Meinig & Partner mbB
(86) International application number: PCT/US2022/047373
(87) International publication number: WO 2023/069677

(56) References cited:
- US-A1- 2006 052 747
- US-A1- 2010 160 711
- US-A1- 2014 257 233
- US-A1- 2016 008 534

## Description

### FIELD

The present disclosure relates generally to compositions for injection to a patient, methods of preparation and use thereof,
said methods and uses not forming part of the invention but being provided as background information,
and devices comprising such compositions.

### BACKGROUND

Numerous men are diagnosed with prostate cancer each year. Traditionally, treatment options include interstitial implant therapy, surgery, and external beam radiotherapy. While the best treatment is still debatable, side effects of treating prostate cancer have become less toxic with implant therapy and radiotherapy.

Since the conception of conformal radiotherapy, physicians have paid attention to the delivered dose to the target and surrounding tissues. Investigators have been able to correlate side effects to the amount of tissue receiving a certain radiation dose. And yet, time, distance, and shielding affect the dose that is delivered. The less time an area is exposed to radiation, the less dose delivered. The greater the distance from the radiation, the less dose delivered.

Current systems provide filler material to treatment sites to decrease the radiation dose to the rectum during radiotherapy for prostate cancer. However, the system that mixes the filler material *in vitro* includes numerous subcomponents, is complex to assemble, and rife with filler mixing errors prior to delivery within a patient at a treatment site. During the foregoing procedures, such errors and mishaps lead unnecessarily to patient risk, increased procedure time, and increased procedure costs. The solution of this disclosure resolves these and other issues of the art.

Document US 2014/257233 A1 describes an adjustable volume syringe which includes a delivery syringe, a reservoir syringe located at least in part within the delivery syringe, and a reservoir plunger located at least in part within the reservoir syringe. The delivery syringe is configured to contain a first amount of a fluid. The reservoir syringe is configured to contain a second amount of the fluid. An administration amount of fluid may be determined at or prior to an administration time. An amount of fluid in the delivery syringe may be adjusted to an administration amount at or prior to the administration time based on whether the administration amount is greater than, less than or equal to the first amount. The administration amount of fluid may be dispensed to a patient at the administration time.

### SUMMARY

The invention provides a system according to claim 1. Embodiments of the invention are defined in the dependent claims. The present disclosure provides also further examples, not falling under the scope of the claims, but provided for better understanding of the invention.

In accordance with certain aspects of the present disclosure, the second constituent is distal of the second cavity and included in a central chamber of the first cavity.

In accordance with certain aspects of the present disclosure, the first constituent is distal of the plunger and included in an outer chamber of the first cavity.

In accordance with certain aspects of the present disclosure, the outer chamber is concentric with a chamber including the second constituent.

In accordance with certain aspects of the present disclosure, a second outer chamber is concentric with the chamber including the second constituent.

In accordance with certain aspects of the present disclosure, the outer chamber runs parallel with a chamber including the second constituent.

In accordance with certain aspects of the present disclosure, the plunger rod is divided into a pair of parallel plunger rods, each of the parallel plunger rods being advanceable within a respective chamber and by a common flange on a proximal end of the plunger rods.

In accordance with certain aspects of the present disclosure, the pair of plunger rods is configured to control the plunger.

In accordance with certain aspects of the present disclosure, the plunger is positioned between an outer surface of the second cavity and an inner surface of the first cavity.

In accordance with certain aspects of the present disclosure, the second cavity includes a third constituent mixable with the second constituent to form a precursor solution.

In accordance with certain aspects of the present disclosure, the second and third constituents are separated by a barrier. Moving the plunger rod and/or the second cavity with respect to the first cavity causes the barrier to open so the third constituent bypasses the barrier and mixes with the second constituent to form the precursor solution.

In accordance with certain aspects of the present disclosure, the second and third constituents are separated by a barrier. Moving the plunger rod and/or the second cavity with respect to the first cavity causes the barrier to toggle to an open state so the third constituent mixes with the second constituent to form the precursor solution.

In accordance with certain aspects of the present disclosure, the barrier is a floating plunger and the distal end of the second cavity includes an internally positioned rib. In some aspects, as the plunger rod is advanced distally, the floating plunger advances distally and contacts the rib to develop a moment causing the floating plunger to tilt and break a seal so constituent within the second cavity can advance distal of the floating plunger.

In accordance with certain aspects of the present disclosure, in a first state, moving the plunger rod causes a third constituent of the second cavity to be delivered through a barrier to mix with the second constituent to form a first mixture (e.g., a precursor solution). In a second state, moving the plunger rod and/or the second cavity causes the mixture and the first constituent to be advanced through respective ports.

In accordance with certain aspects of the present disclosure, the plunger is a gasket or a septum.

In accordance with certain aspects of the present disclosure, a method is disclosed for producing a mixture with any mixing system of this disclosure to deliver to a treatment site. The method can include advancing a plunger rod through the second cavity to open a barrier between the second cavity and the first cavity thereby mixing the second and third constituents to form a first mixture; and moving the second cavity relative to the first cavity causing the first constituent and the first mixture to expel from the plurality of ports to mix together and form the mixture.

In accordance with certain aspects of the present disclosure, an upper proximal end of the first cavity includes a first flange and an upper proximal end of the second cavity includes a second flange incapable of distally advancing beyond the first flange.

In accordance with certain aspects of the present disclosure, the method can include positioning the second constituent distal of the second cavity and in a central chamber of the first cavity.

In accordance with certain aspects of the present disclosure, the method can include positioning the first constituent distal of the plunger and in an outer chamber of the first cavity.

In accordance with certain aspects of the present disclosure, the outer chamber is concentric with a chamber including the second constituent.

In accordance with certain aspects of the present disclosure, the method can include positioning a second outer chamber concentric with the second chamber, the second outer chamber including air.

In accordance with certain aspects of the present disclosure, the method can include orienting the outer chamber parallel with a chamber including the second constituent.

In accordance with certain aspects of the present disclosure, the method can include dividing the plunger rod into a pair of parallel plunger rods; and controlling the plunger by advancing each of the parallel plunger rods within a respective chamber and by a common flange on a proximal end of the pair of parallel plunger rods.

In accordance with certain aspects of the present disclosure, the method can include positioning the plunger is between an outer surface of the second cavity and an inner surface of the first cavity.

In accordance with certain aspects of the present disclosure, the method can include separating the second and third constituents by the barrier; and wherein the barrier opening causes the third constituent to bypass the barrier and form the first mixture.

In accordance with certain aspects of the present disclosure, the method can include separating the second and third constituents by the barrier. In some aspects, the barrier is opened by toggling to an open state so the third constituent mixes with the second constituent to form the first mixture.

To the accomplishment of the foregoing and related ends, certain illustrative aspects are described herein in connection with the following description and the appended drawings. These aspects are indicative, however, of but a few of the various ways in which the principles of the claimed subject matter may be employed. The scope of the invention is defined by the claims.

Other advantages and novel features may become apparent from the following detailed description when considered in conjunction with the drawings.

### BRIEF DESCRIPTION OF THE FIGURES

The accompanying drawings, which are incorporated in and constitute a part of this specification, illustrate various exemplary aspects of the disclosure, and together with the description serve to explain the principles of the present disclosure.
Figs. 1A-1B depict the prostate, rectum, and Denonvilliers' space between the prostate and rectum.
Fig. 2A shows a side plan view of an exemplary mixing system in accordance with certain aspects of the present disclosure.
Fig. 2B shows an upper plan, cross-section view of the exemplary mixing system of Fig. 2A in accordance with certain aspects of the present disclosure.
Fig. 3A depicts an example step in a method using the example mixing system of Figs. 2A-2B, in accordance with certain aspects of the present disclosure.
Fig. 3B depicts an example step in a method using the example mixing system of Figs. 2A-2B, in accordance with certain aspects of the present disclosure.
Fig. 4A depicts an example step in a method using the example mixing system of Figs. 2A-2B, in accordance with certain aspects of the present disclosure.
Fig. 4B depicts an example step in a method using the example mixing system of Figs. 2A-2B, in accordance with certain aspects of the present disclosure.
Fig. 4C depicts an example step in a method using the example mixing system of Figs. 2A-2B, in accordance with certain aspects of the present disclosure.
Fig. 4D depicts an example step in a method using the example mixing system of Figs. 2A-2B, in accordance with certain aspects of the present disclosure.
Figs. 5A-5C depict example steps in a method of priming an example connector, in accordance with certain aspects of the present disclosure.
Fig. 6A shows a side plan view of an exemplary mixing system in accordance with certain aspects of the present disclosure.
Fig. 6B shows an upper plan, cross-section view of the exemplary mixing system of Fig. 6A in accordance with certain aspects of the present disclosure.
Fig. 7A depicts an example step in a method using the example mixing system of Figs. 6A-6B, in accordance with certain aspects of the present disclosure.
Fig. 7B depicts an example step in a method using the example mixing system of Figs. 6A-6B, in accordance with certain aspects of the present disclosure.
Fig. 7C depicts a close-up of the circular section of Fig. 7B, in accordance with certain aspects of the present disclosure.
Fig. 7D depicts an example step in a method using the example mixing system of Figs. 6A-6B, in accordance with certain aspects of the present disclosure.
Fig. 8A depicts an example step in a method using the example mixing system of Figs. 6A-7D, in accordance with certain aspects of the present disclosure.
Fig. 8B depicts an example step in a method using the example mixing system of Figs. 6A-7D, in accordance with certain aspects of the present disclosure.
Fig. 9A depicts an example step in a method using the example mixing system of Figs. 6A-6B, in accordance with certain aspects of the present disclosure.
Fig. 9B depicts an example step in a method using the example mixing system of Figs. 6A-7D, in accordance with certain aspects of the present disclosure.
Fig. 9C depicts an example step in a method using the example mixing system of Figs. 6A-7D, in accordance with certain aspects of the present disclosure.
Fig. 9D depicts an example step in a method using the example mixing system of Figs. 6A-7D, in accordance with certain aspects of the present disclosure.
Fig. 10A shows a side plan view of an exemplary mixing system in accordance with certain aspects of the present disclosure.
Fig. 10B shows an upper plan, cross-section view of the exemplary mixing system of Fig. 10A in accordance with certain aspects of the present disclosure.
Fig. 10C shows a close-up, side, cross-section view of the exemplary mixing system of Fig. 10A in accordance with certain aspects of the present disclosure.
Fig. 11A depicts an example step in a method using the example mixing system of Figs. 10A-10C, in accordance with certain aspects of the present disclosure.
Fig. 11B depicts an example step in a method using the example mixing system of Figs. 10A-10C, in accordance with certain aspects of the present disclosure.
Fig. 12A depicts an example step in a method using the example mixing system of Figs. 10A-10C, in accordance with certain aspects of the present disclosure.
Fig. 12B depicts an example step in a method using the example mixing system of Figs. 10A-10C, in accordance with certain aspects of the present disclosure.
Fig. 12C depicts an example step in a method using the example mixing system of Figs. 10A-10C, in accordance with certain aspects of the present disclosure.
Fig. 13 depicts a partial cross-section view of an example connector and needle attached to an example mixing system of Figs. 10A-10C, in accordance with certain aspects of the present disclosure.
Fig. 14 depicts a flow diagram of a method of using a mixing system according to certain aspects of this disclosure.

### DETAILED DESCRIPTION

Particular aspects of the present disclosure are described in greater detail below.

Particular aspects of the present disclosure may repeat reference numerals and/or letters in the various examples. This repetition is for the purpose of simplicity and clarity and does not in itself dictate a relationship between the various embodiments and/or configurations discussed. Different embodiments may have different advantages, and no particular advantage is necessarily required of any embodiment.

As used herein, the terms "comprises," "comprising," or any other variation thereof are intended to cover a non-exclusive inclusion, such that a process, method, composition, article, or apparatus that comprises a list of elements does not include only those elements, but may include other elements not expressly listed or inherent to such process, method, composition, article, or apparatus. The term "exemplary" is used in the sense of "example" rather than "ideal."

As used herein, the singular forms "a," "an," and "the" include plural reference unless the context dictates otherwise.

As used herein, "approximately" and "about" refer to being nearly the same as a referenced number or value. As used herein, the terms "approximately" and "about" should be understood to encompass ± 10% of a specified amount or value (e.g., "about 90%" can refer to the range of values from 81% to 99%).

As used herein, "operator" can include a doctor, surgeon, or any other individual or delivery instrumentation associated with delivery or use of a mixing system as such systems are described throughout this disclosure.

The compositions herein may be used in various medical procedures, including but not limited to injected to create additional space between the rectum and prostate during treatment, for example in the Denonvilliers' space, thereby reducing rectal radiation dose and associated side effects. Certain embodiments of the disclosure include placing a filler between the radiation target tissue and other tissues. The filler can be a gel composition that increases the distance between the target tissue and other tissues so that the other tissues receive less radiation.

It is understood that "Denonvilliers' space" is a region located between the rectum and prostate. Certain embodiments provide a method of displacing a tissue to protect the tissue against the effects of a treatment involving radiation or cryotherapy. One embodiment involves using a filler mixed by a mixing system of this disclosure to displace the tissue relative to a tissue that is to receive the treatment. Another embodiment involves introducing a filler mixed by a mixing system of this disclosure to displace a first tissue and radiating a second tissue, particularly a second tissue that is close to the first tissue. In another embodiment, the method includes the steps of injecting a filler into a space between tissues; and may further include irradiating one of the tissues so that the other tissue receives less radiation than it would have in the absence of the filler.

Certain embodiments also provide non-claimed methods for treating a tissue of a body by radiation. In one embodiment, the method includes the steps of injecting an effective amount of a filler into a space between a first tissue (e.g., prostate) of a body and a second tissue (e.g., rectum), which can be a critically sensitive organ; and treating the first tissue by radiation whereby the filler within the space reduces passage of radiation into the second tissue. Tissue is a broad term that encompasses a portion of a body: for example, a group of cells, a group of cells and interstitial matter, an organ, a portion of an organ, or an anatomical portion of a body, e.g., a rectum, ovary, prostate, nerve, cartilage, bone, brain, or portion thereof.

The gel of the filler can include polymeric materials which are capable of forming a hydrogel may be utilized. In one embodiment, the polymer forms a hydrogel within the body. A hydrogel is defined as a substance formed when an organic polymer (natural or synthetic) is cross-linked via covalent, ionic, or hydrogen bonds to create a three-dimensional open-lattice structure which entraps water molecules to a gel. Naturally occurring and synthetic hydrogel forming polymers, polymer mixtures, and copolymers may be utilized as hydrogel precursors.

In some aspects, the hydrogel can be formed by a composition formed by constituents (e.g., mixing accelerant fluid, diluent, and PEG together) and may include one or more polysaccharide compounds or a salt thereof. For example, the composition may include a cellulose compound such as carboxymethyl cellulose (CMC) or salt thereof (e.g., CMC) sodium, xanthan gum, alginate or a salt thereof (e.g., calcium alginate, such as Ca-alginate beads), chitosan, and/or hyaluronic acid. In some examples, the composition may comprise a mixture of hyaluronic acid and CMC, and/or may be cross-linked with a suitable crosslinking compound, such as butanediol diglycidyl ether (BDDE). In some aspects, the polysaccharide may be a homopolysaccharide or a heteropolysaccharide

The present disclosure also provides mixing systems to form the gel composition and corresponding medical devices for use and/or delivery to a treatment site of a patient. According to some aspects of the present disclosure, the mixing system may include a plurality of reservoirs with respective lumens. Collectively, the lumens therein may serve as a container for constituents to mix the gel composition of this disclosure. Suitable reservoirs may include, for example, syringes (e.g., a syringe barrel compatible with a manual or automatic injection system) and other fluid containers configured for use with a suitable injection needle. Exemplary materials suitable for the reservoir include, but are not limited to, cyclic olefin polymer, polypropylene, polycarbonate, polyvinyl chloride, and glass. In some aspects, one of these materials (e.g., cyclic olefin copolymer specifically) can have a coating applied to it, such as SiO₂), which is advantageous so the coating can perform as a primary oxygen barrier, behave as a glass-like layer, and can be applied using a vapor deposition process.

According to some aspects of the present disclosure, the compositions may include at least one accelerant (e.g., an activating agent) combined with a precursor mixed from a diluent (e.g., mostly water) and polyethylene glycol (PEG). In some examples, the composition may be or include a gel with a desired gel strength and/or viscosity, such as a biocompatible gel suitable for injection (e.g., through a needle).

The hydrophilic polymer can be any gelling agent(s), including natural ones or synthetic in origin, and may be anionic, cationic, or neutral. Non-limiting examples of the gelling agents include polysaccharides such as gellan gum, xanthan gum, gum arabic, guar gum, locust bean gum, alginate, and carrageenans.

The concentrations of gelling agent(s) in the composition described in this disclosure may range from about 0.01% to about 2.0% by weight with respect to the total weight of the composition, such as from about 0.02% to about 1.5%, from about 0.05% to about 1.0%, from about 0.05% to about 0.50%, from 0.05% to about 0.15%, from about 0.10% to about 0.20%, from about 0.15% to about 0.25%, from about 0.20% to about 0.30%, from about 0.25% to about 0.35%, from about 0.30% to about 0.40%, from about 0.35% to about 0.45%, from about 0.40% to about 0.50%, from about 0.1% to about 0.5%, or from about 0.1% to about 0.15% by weight with respect to the total weight of the composition. In at least one example, the total concentration of the gelling agent(s) in the composition may range from about 0.05% to about 0.5% by weight with respect to the total weight of the composition.

In some examples, the composition may have a viscosity ranging from about 0.001 Pascal-second (Pa·s) to about 0.100 Pa·s at a shear rate of 130 s⁻¹, such as, e.g., from about 0.005 Pa·s to about 0.050 Pa·s, from about 0.010 Pa·s to about 0.050 Pa·s, from about 0.010 Pa·s to about 0.030 Pa·s, from about 0.010 Pa·s to about 0.020 Pa·s, from about 0.020 Pa·s to about 0.030 Pa·s, or from about 0.020 Pa·s to about 0.040 Pa·s at a shear rate of 130 s⁻¹. Thus, for example, the composition may be or comprise a gel having a viscosity of about 0.005 Pa·s, about 0.006 Pa·s, 0.008 Pa·s, about 0.010 Pa·s, about 0.011 Pa·s, about 0.012 Pa·s, about 0.013 Pa·s, about 0.014 Pa·s, about 0.015 Pa·s, about 0.016 Pa·s, about 0.017 Pa·s, about 0.018 Pa·s, about 0.019 Pa·s, about 0.020 Pa·s, about 0.022 Pa·s, about 0.024 Pa·s, about 0.026 Pa·s, about 0.028 Pa·s, about 0.030 Pa·s, about 0.032 Pa·s, about 0.034 Pa·s, about 0.036 Pa·s, about 0.038 Pa·s, about 0.040 Pa·s, about 0.042 Pa·s, about 0.044 Pa·s, about 0.046 Pa·s, about 0.048 Pa·s, or about 0.050 Pa·s at a shear rate of 130 s⁻¹. In at least one example, the composition may have a viscosity greater than 0.0050 Pa·s at a shear rate of 130 s⁻¹, e.g., a viscosity ranging from about 0.005 Pa·s to about 0.050 Pa·s, at a shear rate of 130 s⁻¹. In at least one example, the composition may have a viscosity greater than 0.010 Pa·s at a shear rate of 130 s⁻¹, e.g., a viscosity ranging from about 0.010 Pa·s to about 0.030 Pa·s, at a shear rate of 130 s⁻¹.

Alternatively or additionally, the composition may have a viscosity ranging from about 0.001 Pa·s to about 0.050 Pa·s at a shear rate of 768 s⁻¹, such as, e.g., from about 0.002 Pa·s to about 0.030 Pa·s, from about 0.003 Pa·s to about 0.020 Pa·s, from about 0.004 Pa·s to about 0.010 Pa·s, from about 0.004 Pa·s to about 0.006 Pa·s, from about 0.005 Pa·s to about 0.007 Pa·s, from about 0.006 Pa·s to about 0.008 Pa·s, from about 0.007 Pa·s to about 0.009 Pa·s, or from about 0.008 Pa·s to about 0.01 Pa·s at a shear rate of 768 s⁻¹. Thus, for example, the composition may be or comprise a gel having a viscosity of about 0.003 Pa·s, about 0.004 Pa·s, about 0.005 Pa·s, about 0.006 Pa·s, about 0.007 Pa·s, about 0.008 Pa·s, about 0.009 Pa·s, or about 0.010 Pa·s at a shear rate of 768 s⁻¹. In at least one example, the composition may have a viscosity less than 0.010 Pa·s at a shear rate of 768 s⁻¹, e.g., a viscosity ranging from about 0.005 Pa·s to about 0.009 Pa·s at a shear rate of 768 s⁻¹. In at least one example, the composition may have a viscosity ranging from about 0.004 Pa·s to about 0.010 Pa·s at a shear rate of 768 s⁻¹. Further, for example, the composition may have a viscosity ranging from about 0.010 Pa·s to about 0.030 Pa·s, e.g., about 0.017 Pa·s at a shear rate of 130 s⁻¹ and a viscosity ranging from about 0.004 Pa·s to about 0.010 Pa·s, e.g., about 0.007 Pa·s, at a shear rate of 768 s⁻¹.

The mixing system herein may include or be removably connected to one or more needles. In some examples, the needle may be a hypodermic needle, and may range from a size of 7 gauge (4.57 mm outer diameter (OD), 3.81 mm inner diameter (ID)) to 33-gauge (0.18 mm OD, 0.08 mm ID), e.g., a size of 16 gauge (1.65 mm OD, 1.19 mm ID), 18 gauge, 21 gauge (0.82 mm OD, 0.51 mm ID), 22 gauge (0.72 mm OD, 0.41 mm ID), 23 gauge (0.64 mm OD, 0.33 ID), or 24 gauge (0.57 mm OD, 0.31 mm ID). Exemplary materials for the needle include, but are not limited to, metals and metal alloys, such as stainless steel and Nitinol, and polymers. The distal tip of the needle may be sharpened, and may have a beveled shape. The proximal end of the needle may include a suitable fitting/adaptor (e.g., a Luer adapter) for engagement with a syringe or other reservoir. In some examples, the needle may include an elongated tube or catheter between the needle tip and the proximal fitting/adapter.

According to some aspects of the present disclosure, the filler compositions herein, e.g., the compositions prepared by the methods herein may have sufficient strength, e.g., gel strength, to withstand the forces and thus minimizing the effects of the forces on the continuity of the three-dimensional gel network. In the meantime, the composition with sufficient strength may have a viscosity suitable for injection, e.g., a viscosity that does not render the composition stuck in the reservoir(s), delivery lumen, or a needle connected therewith.

According to some aspects of the present disclosure, the composition may maintain its three-dimensional structure until the gel is injected through a needle, whereupon the structure may form fragments of the original continuous, three-dimensional network. Those gel fragments may have a diameter corresponding to the diameter of the injection needle, such that the fragments are as large as possible in-vivo to retain as much of the three-dimensional structure of the gel as possible. Injection of these larger-sized particles or fragments is believed to increase the amount of time the gel remains within the tissue.

The amount of force required to move the composition through a needle aperture (generally described as "peak load" force) may depend on the viscosity of the composition, the dimensions of the needle (inner diameter, outer diameter, and/or length), and/or the material(s) from which the needle is formed. For example, a greater amount of force may be applied to inject the composition through a 33-gauge needle in comparison to a 7-gauge needle. Additional factors that may affect the amount of force applied to inject the composition may include the dimensions of a catheter (inner diameter, outer diameter, and/or length) connecting the mixing system to the needle. Suitable peak loads for injection with one or two hands may range from about 5 lbf to about 25 lbf, such as from about 10 lbf to about 20 lbf, e.g., about 15 lbf. The loads measured for a given gel concentration may vary for different needles and flow rates.

According to some aspects of the present disclosure, the size of the needle may be chosen based on the viscosity and/or components of the composition, or vice versa. According to some aspects of the present disclosure, the size of the needle may be 23 gauge or 25 gauge. In some cases, a larger size of 18-gauge, 20 gauge, 21 gauge, or 22 gauge may be used to inject the compositions herein.

According to some aspects of the present disclosure, the mixing system of this disclosure can be included in a kit for introducing a filler into a patient, whereby the filler can include any of the gel compositions of this disclosure. Kits or systems for mixing a gel composition of this disclosure, such as hydrogels, may be prepared so that the precursor(s) and any related activating agent(s) are stored in the kit with diluents as may be needed. Applicators may be used in combination with the same. The kits can be manufactured using medically acceptable conditions and contain components that have sterility, purity and preparation that is pharmaceutically acceptable. Solvents/solutions may be provided in the kit or separately. The kit may include syringes and/or needles for mixing and/or delivery. The kit or system may comprise components set forth herein.

During some examples of use, once saline has been injected to the treatment site, a mixing system can be connected to a needle (e.g., an 18-gauge spinal needle) to then inject a 5-10 mm layer of filler (e.g., gel composition) along the posterior wall of the prostate between the prostate and rectum. Once the filler has been injected into the space between the rectum and prostate, ultrasound images can be obtained.

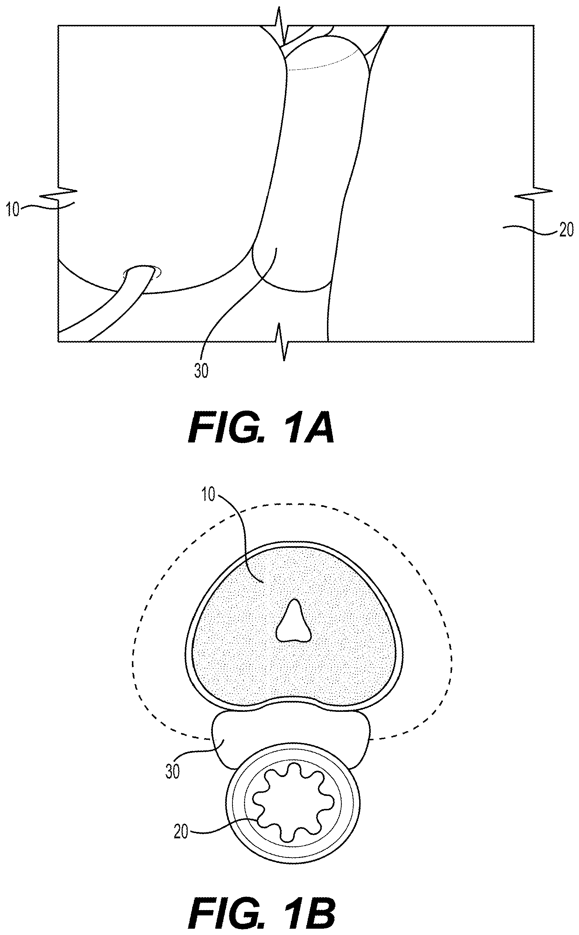

Turning to the drawings, Fig. 1A is a perspective view and Fig. 1B is a partial cross-section view illustrating example filler 30, in the form of a gel composition having been delivered by the mixing system of this disclosure between rectum 20 and prostate 10 of a patient in Denonvilliers' space.

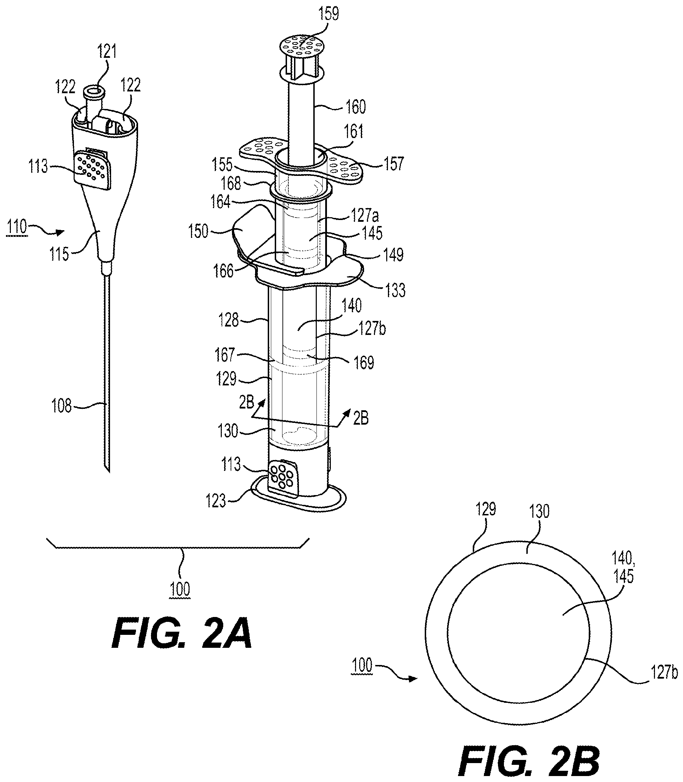

Fig. 2A shows a partially exploded perspective view of an exemplary mixing system 100 in accordance with certain aspects of the present disclosure. System 100 can be used for mixing a mixture for use as filler 30. System 100 can include a first cavity 128 with an open upper proximal end 149. Cavity 128 can be substantially tubular or otherwise elongate with an upper flange 133 at or adjacent end 149.

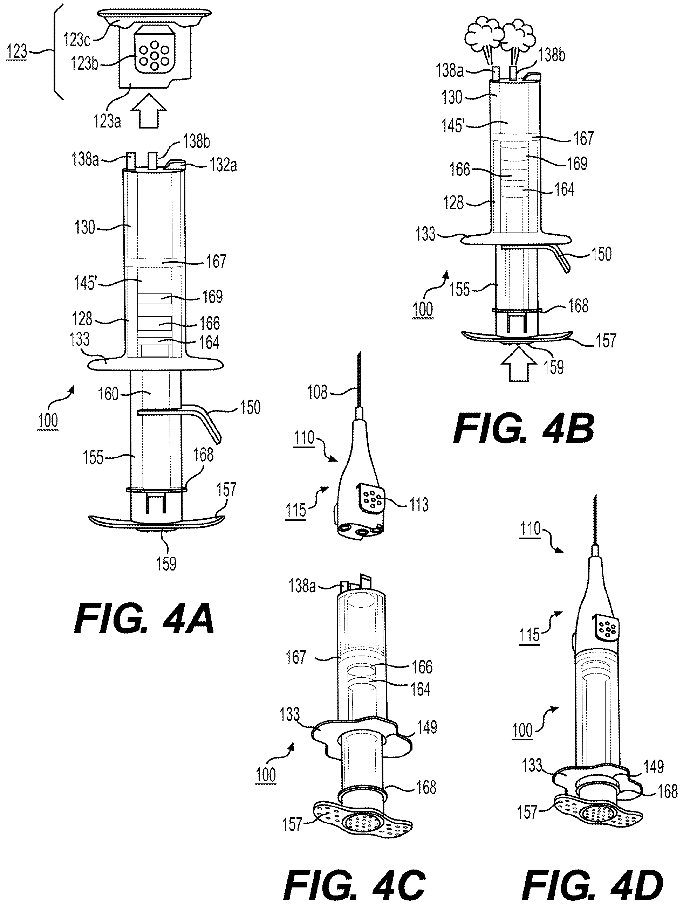

A second cavity 155 can be concentric with and advanceable through end 149 of cavity 128. Cavity 155 can include an open upper proximal end 161. A plunger stopper 167 can be positioned on a distal end of cavity 155 so that cavity 155 can function as a plunger rod to advance fluids stored distal of stopper 167 between an outer surface of cavity 155 and an inner surface of cavity 128. In some aspects, advancing cavity 155 within cavity 128 can cause stopper 167 between the outer surface of cavity 155 and an inner surface of cavity 128 to advance constituents (e.g., constituent 130) from cavity 128 and through at least one of distally positioned fluid ports (e.g., ports 138a, 138b shown in Fig. 4A).

Stopper 167 can be a septum, moveable seal or membrane, or any other feature configured to advance fluids within an enclosed chamber. A plunger rod 160 can be included and advanceable through end 161 and configured to move s constituent 145 (e.g., a fluid such as diluent) from inside cavity 155 and through a distal end. As used herein, the term "fluid" is defined broadly and can include liquids, gels and particulate matter such as granules, pellets, or powders, or any combination of liquids, gels, oils, and/or particulate matter (e.g., granules, pellets, or powders). A plunger stopper 164 can be located at a distal end of rod 160. Cavity 155 can include a fluid chamber 127a distal of stopper 164, which can include constituent 145 (e.g., diluent). The diluent used in systems of this disclosure, including system 100, can be a branched polymer having a plurality of succinimidyl termini dissolved in a low pH (4.0) containing a low molecular weight precursor including nucleophiles, though other diluent fluid solutions are contemplated within the scope of this disclosure.

Rod 160 can be advanced by flange 159 positioned on a proximal end of rod 160. In some examples, flange 159 is shaped and arranged so that it is prevented from advancing distally past end 161. Cavity 155 can be substantially tubular or otherwise elongate with an upper flange 157 at or adjacent end 161. Flange 157 can be arranged and/or shaped so that it is incapable of distally advancing past the flange 133. An annular extrusion 168 can be positioned distal of flange 157 and extended outwardly from cavity 155. Extrusion 168 can be completely annular or only partially or some other shape configured to prevent cavity 155 from advancing distal of end 149. In this respect, once extrusion 168 contacts end 149, distally advancing flange 157 can cause stopper 169 to distally advance precursor 145' and stopper 167 to advance constituent 130 distally through fluid ports of cavity 128. Constituent 130 can be an accelerant that is mixable with precursor 145' to form the mixture of filler 30 (e.g., a gel composition).

A constituent 140 (e.g., a hydrophilic polymer, PEG, etc.,) can be distal of the cavity 155 and housed initially in a fluid chamber 127b of cavity 128. Chamber 127b can be coaxial with chamber 127a but be formed and/or housed in a distal portion of cavity 128. In some aspects, chamber 127b can be concentric with a chamber 129 housing constituent 130 so that chamber 127b runs through chamber 129 to at least one of the ports 138. This is more clearly shown in Fig. 2B, which is a top plan cross-sectional view of section 2B-2B of Fig. 2A showing how chamber 129 can be formed with chamber 127b concentric and internal thereto.

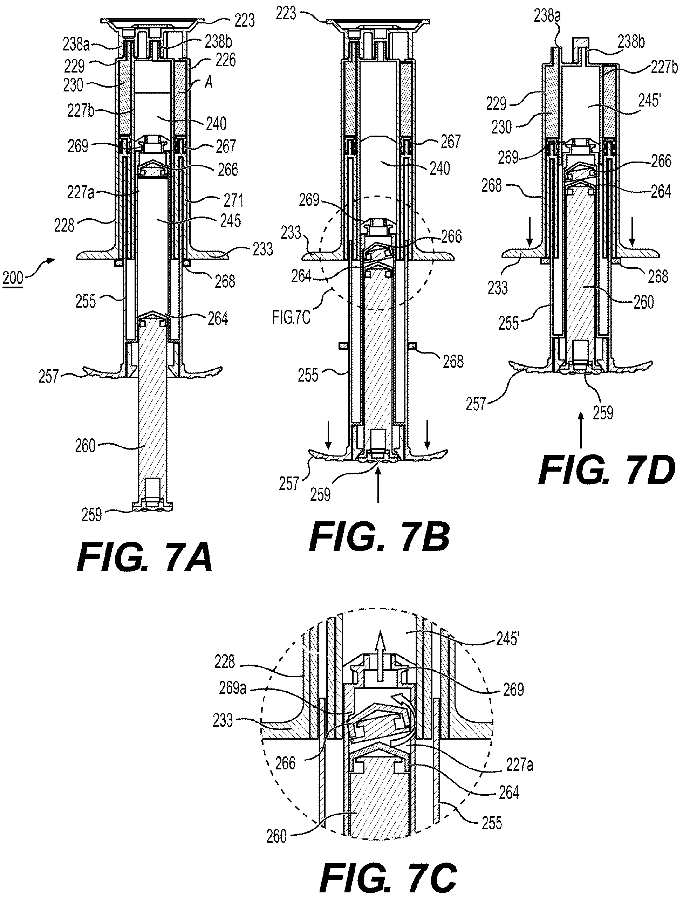

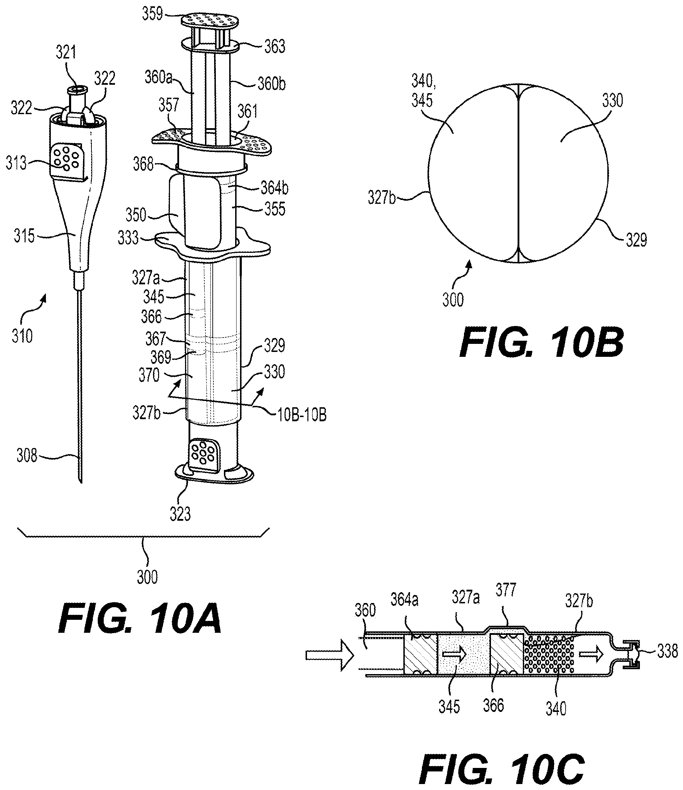

Distally moving rod 160 can cause stopper 164 to advance constituent of chamber 127a so as to open a barrier between chambers 127a, 127b thereby allowing constituents 140, 145 to intermix and form precursor. In some aspects, a floating stopper 166 can be distal of stopper 164 and be configured to toggle when activated by pressure so as to open and allow constituent of chamber 127a to pass. A plunger stopper 169 can be distal of stopper 166 and be configured to urge precursor 145', once formed in chamber 127b, distally through distally positioned fluid ports of cavity 128. Stopper 169 can include a membrane or seal centrally positioned and configured with a one-way valve through which constituents can flow into chamber 127b. Stoppers 164, 166, and 169 is more clearly shown in Fig. 4A after having been caused to open to permit intermixing of constituents 140, 145 distal thereof to form precursor 145'. Non-limiting examples of opening a barrier between chambers 127a, 127b are further illustrated and explained in Figs. 7A - 7D and Fig. 10C though any openable barrier configured to separate constituents is contemplated for use in and between chambers 127a, 127b.

In some example, constituent of chamber 127a can be a constituent 145 (e.g., diluent) and chamber 127b can include constituent 140 (e.g., activating agent, such as PEG or any other agent mixable with diluent to form precursor). The diluent can be a branched polymer having a plurality of succinimidyl termini dissolved in a low pH (4.0) containing a low molecular weight precursor comprising nucleophiles, though other diluent fluid solutions are contemplated within the scope of this disclosure. Once mixed together, precursor 145' can be formed in chamber 127b.

The system 100 can be packaged in a kit that can include a needle assembly 110 with needle 108 attachable to a distal end of system 100 at a connector 115. Needle 108 can be any needle of this disclosure suitable for hydrodissection as well as to the treatment site delivering filler 30 (e.g., the gel composition). A proximal end of needle 108 can be connected to a distal end of connector 115. Connector 115 can include a central lumen 117 running therethrough.

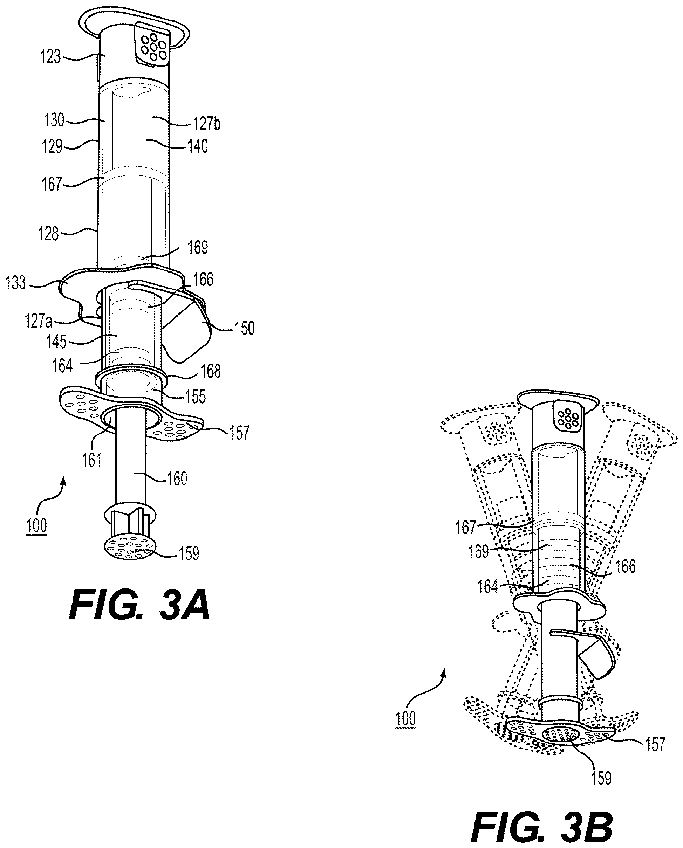

Now, turning to Figs. 3A-4D are example steps of a process of using system 100 according to certain aspects of this disclosure. While certain steps are shown as a sequence between each figure, in other embodiments, fewer steps are contemplated and the order by which steps are performed can be different than what is illustrated. In Fig. 3A, system 100 is introduced in a first state with cap 123 attached to cavity 128 while covering ports 138a, 138b. Cavity 155 and rod 160 are depicted relatively withdrawn from respective open ends 149, 161 while a retainer 150 is positioned between flanges 133, 157. Retainer 150 in the depicted configuration can prevent cavity 155 from distally moving deeper into cavity 128 as a result of retainer 150 being wedged therebetween.

In contrast, as shown in Fig. 3B, rod 160 has been advanced distally so that flange 159 is relatively flush with flange 157 and constituent 145 urged distally by stopper 164. This urging can cause the barrier associated with stopper 166 to toggle or otherwise move allowing constituent 145 to egress from chamber 127a, around or through stopper 169, which is distal of stopper 166, and advance into chamber 127b to mix with constituent 140. Now, with constituents 140, 145 together in chamber 127b, system 100 is shaken back and forth to ensure precursor 145' forms as a result of mixing between constituent 145 and constituent 140, while constituent 130 remains in chamber 129. Preferably, the shaking action of Fig. 3B is done while the ports 138a, 138b are oriented generally upward. However, the shaking to effect proper mixing of precursor 145' can be performed in other port orientations (e.g., generally downward, etc.), as needed or required.

In Fig. 4A, cap is 123 removed revealing ports 138a, 138b. As shown, port 138b can be in fluid communication with in chamber 127b and port 138a can be in fluid communication with chamber 129. Stopper 167 as arranged is configured to move constituent 130 from chamber 129 through port 138a and/or constituent (e.g., precursor 145') within chamber 127b. Each of ports 138a, 138b can include a seal or membrane so as to control egress of constituents from cavity 128 therethrough. In Fig. 4B, with precursor 145' formed and constituent 130 in chamber 129, flange 157 and/or flange 159 can be distally advanced slightly to purge any air A from system 100 out through respective ports 138a, 138b. In some aspects, a user can advance flange 157 causing cavity 155 to advance stopper 167 to advance distally. Advancing flange 157 in this manner can also cause corresponding flange 159 of rod 160 to advance in tandem with flange 157 so that air inside chamber 127b or cavity 128 can similarly be expelled. Retainer 150 can be positioned with respect to cavity 155 so as to contact flange 133 and prevent unwanted or otherwise unintended delivery of constituents from cavity 128 out through ports 138a, 138b. Preferably, air A is purged as shown while system 100 is oriented generally upward.

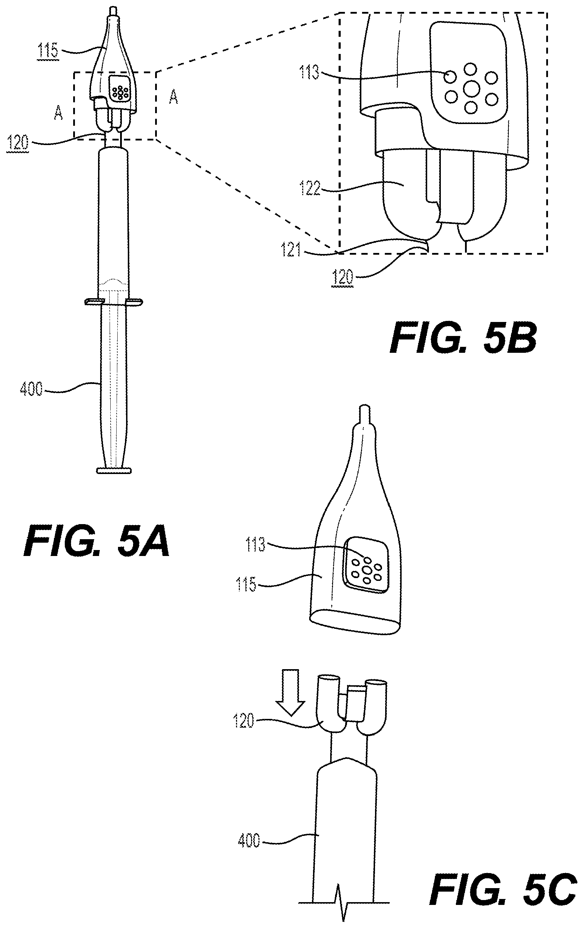

In Fig. 4C, with air A purged, system 100 is now being connected to connector 115, which can similarly be primed and connected to needle 108 at the treatment site. In Fig. 4D, system 100 is now connected to connector 115. Aspects of priming connector 115 are discussed more in Figs. 5A-5C.

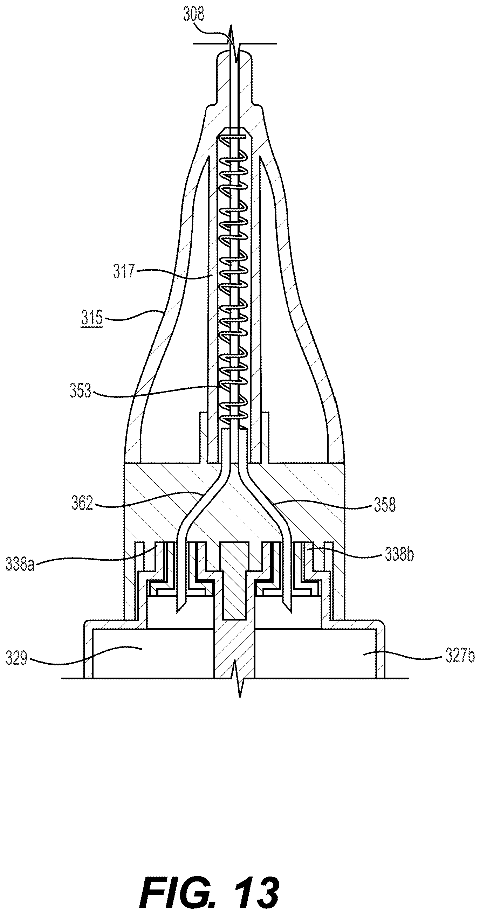

With system 100 ready and connector 115 primed, a user in Fig. 4D can advance flange 157 distally so that corresponding cavity 155 and rod 160 distally drive respective stoppers167, 169, and advance precursor 145' from chamber 127b and constituent 130 from chamber 129, through ports 138a, 138b, and into connector 115. As long as flange 157 continues advancing, precursor 145' and constituent 130 can mix within a central lumen of connector 115 and continue egressing through needle 108 and ultimately to the treatment site. It can be seen that extrusion 168 prevents flange 157 from distally advancing past flange 133 on account of extrusion 168 being incapable of advancing distal of end 149. Optionally, a central lumen of connector 115 (e.g., lumen 317 of Fig. 13) can include a static mixer configured to thoroughly mix the constituents together to form the mixture to be delivered to the treatment site. System 100 as shown is relatively easy to assemble and minimizes potential unintentional gel mixing errors prior to delivery.

Separately, in Fig. 5A, connector 115 is shown connected to syringe 400 via adaptor 120, as illustrated more clearly in Fig. 5B, which is a close-up of adaptor 120 coupling to connector 115. While not shown, connector 115 and syringe 400 together are contemplated to be connected to needle 108 for hydrodissection at the treatment site with saline from syringe 400. After hydrodissection with syringe 400, needle 108 and connector 115 can be released from syringe 400, as shown in Fig. 5C, so that primed connector 115 and needle 108 can then be connected to system 100, as in Figs. 4C to Fig. 4D. Adaptor 120 can provide a fluid bridge 122, 121 from internal lumen of connector 115 to syringe 400. Connector 115 can include an externally positioned button 113 to open and close corresponding connecting latches of connector 115. In this respect, adaptor 120 can include one or more latches configured to removably attach to connector 115 via button 113. However, other coupling approaches between connector 115, adaptor 120, and syringe 400 are contemplated as needed or required. For example and without limitation, snap fit connectors, magnetic connectors, female - male connectors, hook and loop fasteners and the like are contemplated.

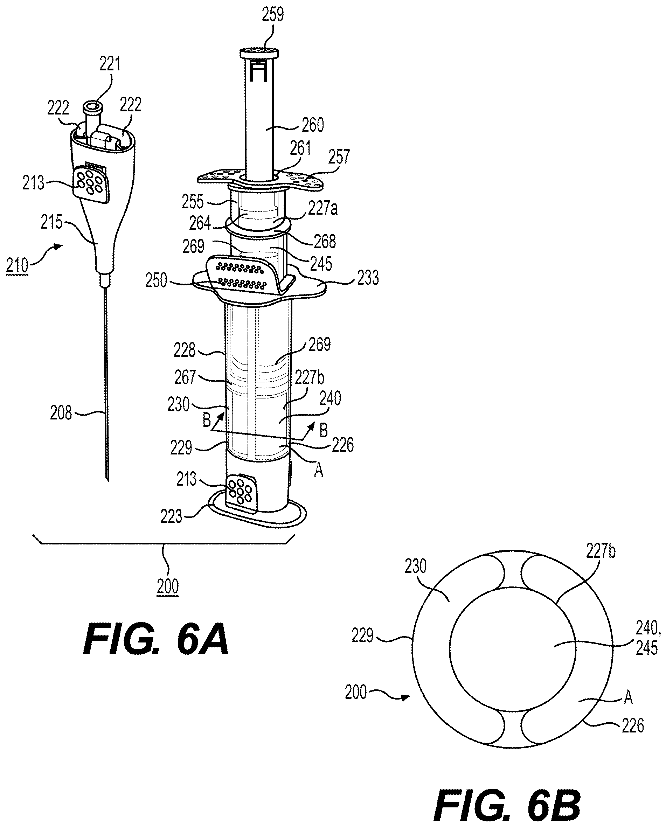

Turning to Figs. 6A-6B, another exemplary mixing system 200 is shown in accordance with certain aspects of the present disclosure for mixing a mixture for use as filler 30. Here, chamber 227b can be coaxial with chamber 227a but be formed and/or housed in a distal portion of cavity 228. In some aspects, chamber 227b can be concentric with chamber 229 housing constituent 230 so that chamber 227b runs through chamber 229 to at least one of the ports 238a, 238b. Rather than being completely tubular and surrounding all of chamber 227b, chamber 229 can be semi-tubular (e.g., hemispherical, crescent shaped, etc.). Cavity 228 can include an air chamber 226 through which chamber 227b can also run through and be at least partially concentric, as more clearly shown in Fig. 6B, which is a top plan cross-sectional view of section B-B of Fig. 6A. Specifically, Fig. 6B shows chambers 229, 226 formed about chamber 227b concentric and internal thereto. In some aspects, chambers 226 and 229 can include the same inner and outer diameters and be tubular halves or portions positioned on opposite sides of chamber 227. Stopper 267 of system 200 can be correspondingly shaped so as to accommodate and control constituents in chamber 229.

In Figs. 7A-7D, side plan cross sectional views of system 200 are shown. In particular, Fig. 7A shows system 200 in a first state with rod 260 fully withdrawn and constituent 245 stored in chamber 227a proximal of stopper 266 and chamber 227b. In Fig. 7B, an upward force can be applied to flange 259 while an opposite oriented force can be applied to flange 257. This push-pull action can cause stopper 266 to toggle, as shown more clearly in Fig. 7C which is a close-up stopper 266 being opened in Fig. 7B. As can be seen in Fig. 7C, a distal tip of stopper 266 is being advanced into a proximal end of stopper 269 at rib or protrusion 269a causing a toggling action by stopper 266. In some aspects, contacting rib or protrusion 269a can cause stopper 264 to develop a moment causing the floating stopper 266 to tilt and break its seal so as to permit flow to advance distal thereof. For example, once toggled, the denoted arrows show a flow path of constituent from chamber 227a around or about toggled stopper 266 and through an opening in stopper 269.

As this happens in certain aspects, cavity 255 can be urged proximally by the force applied to flange 257 so as to open the seal of stopper 269. In other examples, it is contemplated that instead of a distal tip of stopper 266 or a proximal end of rib or protrusion 269a of stopper 269 being shaped to induce toggling, achieving a predetermined pressure can induce toggling or otherwise break a seal associated with stopper 269 and/or stopper 266 to actuate flow of constituents between and/or through chambers 227a, 227b.

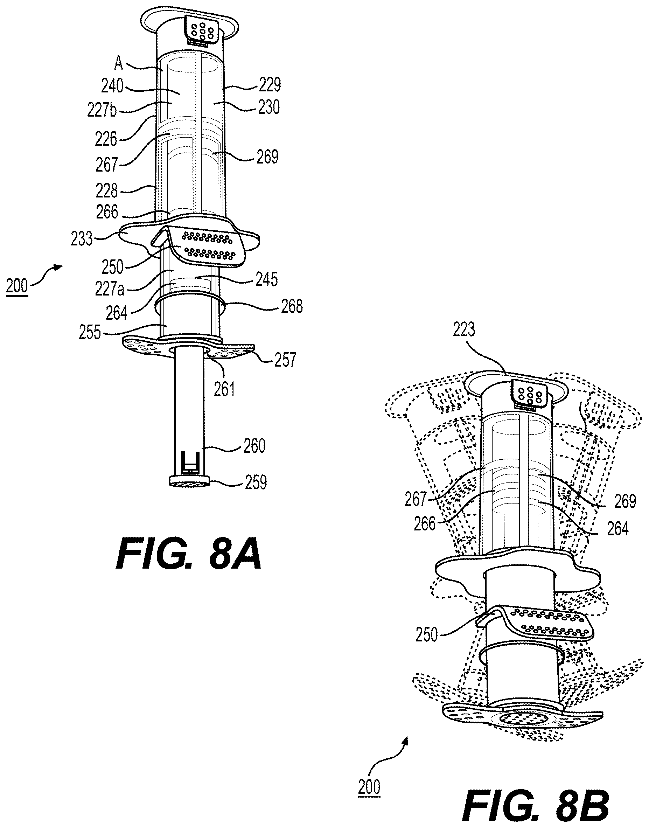

In Fig. 7D, with cap 223 removed and precursor 245' formed in chamber 227b, an upward force is continued being applied to flanges 259 and 257 while an opposite oriented force is applied by a user to flange 233. In turn, cavity 255 is caused to advance stopper 267 within cavity 228 so as to advance constituent 230 and stopper 269 advancing precursor 245' simultaneously from system 200 and egress out through ports 238a, 238b, as shown more particularly in Figs. 8A-9D.

Turning to Figs. 8A-9D are example steps of a process of using system 200 according to certain aspects of this disclosure. While certain steps are shown as a sequence between each figure, in other embodiments, fewer steps are contemplated and the order by which steps are performed can be different than what is illustrated. In Fig. 8A, system 200 is introduced in a first state with cap 223 attached to cavity 228 while covering ports 238a, 238b. Cavity 255 and rod 260 are withdrawn from respective open ends 249, 261 while a retainer 250 positioned between flanges 233, 257. Retainer 250 in the depicted configuration can prevent cavity 255 from distally moving distally into cavity 228 as a result of retainer 250 being wedged between flanges 233, 257.

In contrast, as shown in Fig. 8B, rod 260 has been advanced distally so that flange 259 is relatively flush with flange 257 and constituent 245 has been moved by stopper 264. Similar to system 100, this urging can cause the barrier associated with stopper 266 to toggle or otherwise move allowing constituent 245 to egress from chamber 227a and advance through stopper 266 into chamber 227b to mix with constituent 240. Now, system 200 is shaken back and forth to ensure precursor 245' forms as a result of mixing between constituent 245 and constituent 240, while constituent 230 remains in chamber 229. Preferably, the shaking action of Fig. 8B is done while the ports 238a, 238b are oriented generally upward. However, the shaking to effect proper mixing of precursor 245' can be performed in other port orientations (e.g., generally downward, etc.), as needed or required.

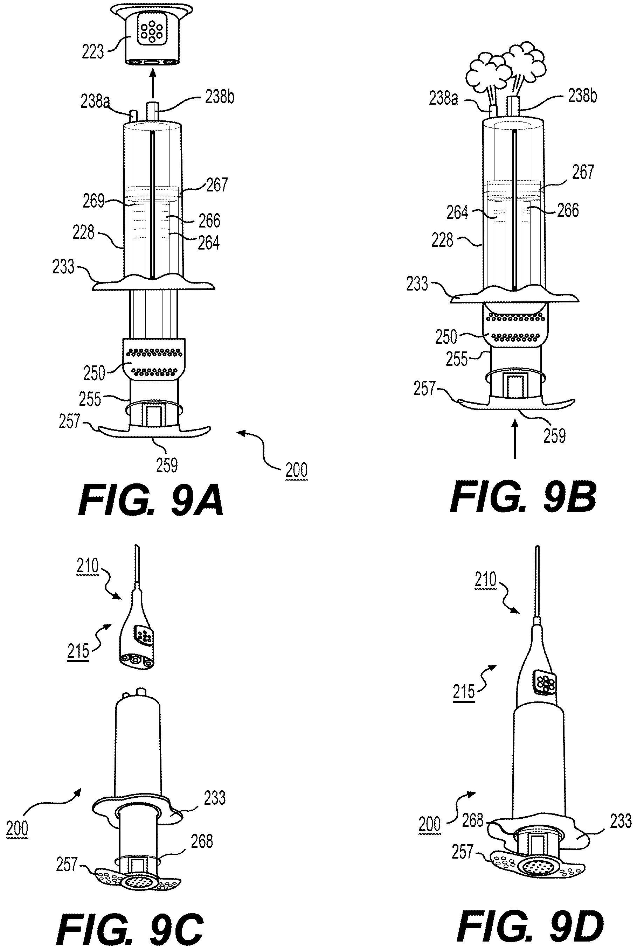

In Fig. 9A, cap is 223 removed revealing ports 238a, 238b. In Fig. 9B, with precursor 245' formed and constituent 230 in chamber 229, flange 257 and/or flange 259 can be distally advanced slightly to purge any air A from system 200 out through respective ports 238a, 238b. In some aspects, a user can advance flange 257 causing cavity 255 to advance stopper 267 to advance distally and cause corresponding flange 259 of rod 260 to advance in tandem with flange 257 so that air inside chamber 227b or cavity 228 can similarly be expelled. Retainer 250 can be positioned with respect to cavity 255 so as to contact flange 233 and prevent unwanted or otherwise unintended delivery of constituents from cavity 228 out through ports 238. Preferably, air A is purged as shown while system 200 is oriented generally upward.

In Fig. 9C, with air A purged, system 200 is now being connected to connector 215, which can be primed and connected to needle 208 at the treatment site. In Fig. 9D, system 200 is now connected to connector 215. With system 200 ready and connector 215 primed, a user in Fig. 9D can advance flange 257 distally so that corresponding cavity 255 and rod 260 distally drive respective stoppers 267, 269, and advance precursor 245' from chamber 227b and constituent 230 from chamber 229, through ports 238a, 238b, and into connector 215. As long as flange 257 continues advancing, precursor 245' and constituent 230 can mix within a central lumen of connector 215 and continue egressing through needle 208 and ultimately to the treatment site. It can be seen that extrusion 268 prevents flange 257 from distally advancing past flange 233 on account of extrusion 268 being incapable of advancing distal of end 249. System 200 as shown is relatively easy to assemble and minimizes potential unintentional gel mixing errors prior to delivery.

Turning to Figs. 10A-10C, another exemplary mixing system 300 is shown in accordance with certain aspects of the present disclosure for mixing a mixture for use as filler 30. Here, chamber 327b can be coaxial with chamber 327a but be formed and/or housed in a distal portion of cavity 328. In some aspects, chamber 327b can be concentric with chamber 329 housing constituent 330 so that chamber 327b runs through chamber 329 to at least one of the ports 338a, 338b. Rather than being completely tubular and surrounding all of chambers 327b, 329 can run parallel with chamber 327b. For example, an inner surface of each can be planar, relatively flush with the other, with an outer surface being hemispherical or otherwise curved so together, chambers 327b, 329 can together form a tubular shape. Chambers 327b, 329 can be semi-tubular (e.g., hemispherical, crescent shaped, etc.,), as more clearly shown in Fig. 10B, which is a top plan cross-sectional view of section 10B-10B of Fig. 10A. In some aspects, chambers 327b, 329 can include the same inner and outer diameters and be tubular halves or portions positioned on opposite sides of cavity 328. Stopper 367 of system 300 can be correspondingly shaped so as to accommodate and control constituents in some or all of chamber 329.

Rod 360 can also be divided into a pair of parallel plunger rods 360a, 360b. Rod 360a can be configured to slide within and control fluid in chambers 327a, 327b. Rod 360a can include stopper 364a configured to advance constituent 345 from chamber 327a and into chamber 327b, similar to previous stoppers 264, 164. Rod 360b can be configured to slide within chamber 329 and similarly include stopper 364b. Rods 360a, 360b can share a common flange 359 and be advanceable within respective chambers of cavity 355.

Turning to Fig. 10C, a partial cross section view of an optional embodiment is shown with stoppers 364a, 366 when a barrier between chambers 327a, 327b is being opened. It is understood that the depicted embodiment is an alternative approach to a toggleable barrier associated with previously described examples (e.g., stopper 266). As such, Fig. 10C does not use or otherwise require use of stopper 369 as shown, since cavity 355 includes a portion 377 with an outer diameter greater than chambers 327a, 327b. In this respect, a barrier associated with stopper 366 can be opened by advancing stopper 364a distally causing constituent 345 to advance stopper 366 until it is at or adjacent portion 377. Once so positioned, a space between portion 377 and stopper 366 allows constituent 345 to flow around or through stopper 366 and into chamber 327b to mix with constituent 340.

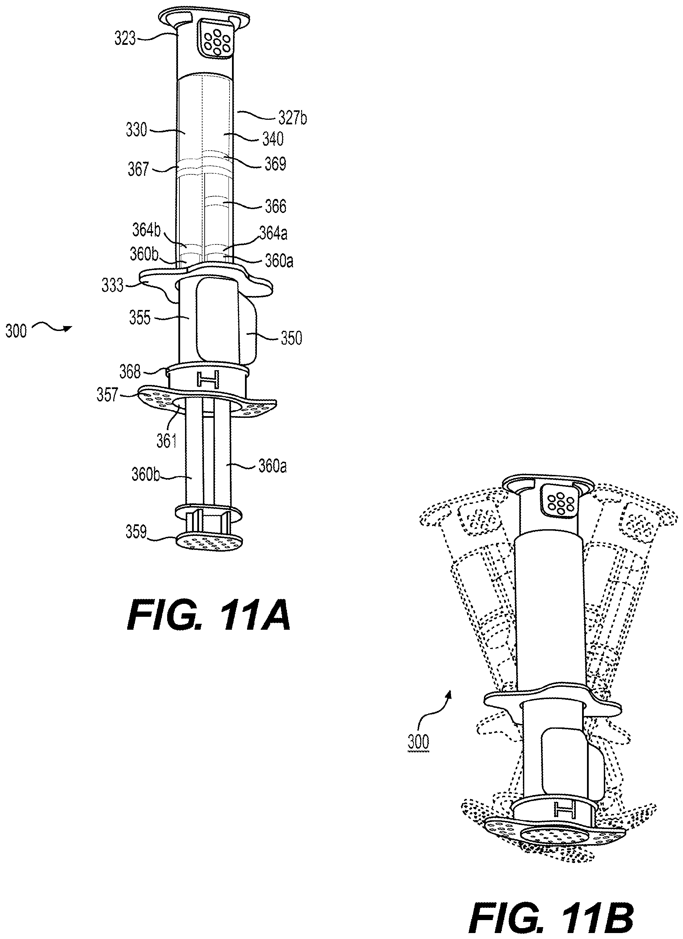

Turning to Figs. 11A-12C are example steps of a process of using system 300 according to certain aspects of this disclosure. While certain steps are shown as a sequence between each figure, in other embodiments, fewer steps are contemplated and the order by which steps are performed can be different than what is illustrated. In Fig. 11A, system 300 is introduced in a first state with cap 323 attached to cavity 328 while covering ports 338a, 338b. Cavity 355 and rods 360a, 360b are depicted relatively withdrawn from respective open ends 349, 361 while a retainer 350 is positioned between flanges 333, 357. Retainer 350 in the depicted configuration can prevent cavity 355 from distally moving into cavity 328 as a result of retainer 350 being wedged between flanges 333, 357.

In contrast, as shown in Fig. 11B, rods 360a, 360b have been advanced distally so that constituent 345 has been moved distally by stopper 364. This urging can cause the barrier associated with stopper 366 to toggle, open, or bypass, as discussed in Fig. 10C, allowing constituent 345 to egress from chamber 327a, around or through stopper 369, which is distal of stopper 366, and advance into chamber 327b to mix with constituent 340. Now, with constituents 340, 345 together in chamber 327b, system 300 is shaken back and forth to ensure precursor 345' forms as a result of mixing between constituent 345 and constituent 340, while constituent 330 remains in chamber 329. Preferably, the shaking action of Fig. 11B is done while the ports 338a, 338b are oriented generally upward. However, the shaking to effect proper mixing of precursor 345' can be performed in other port orientations (e.g., generally downward, etc.), as needed or required.

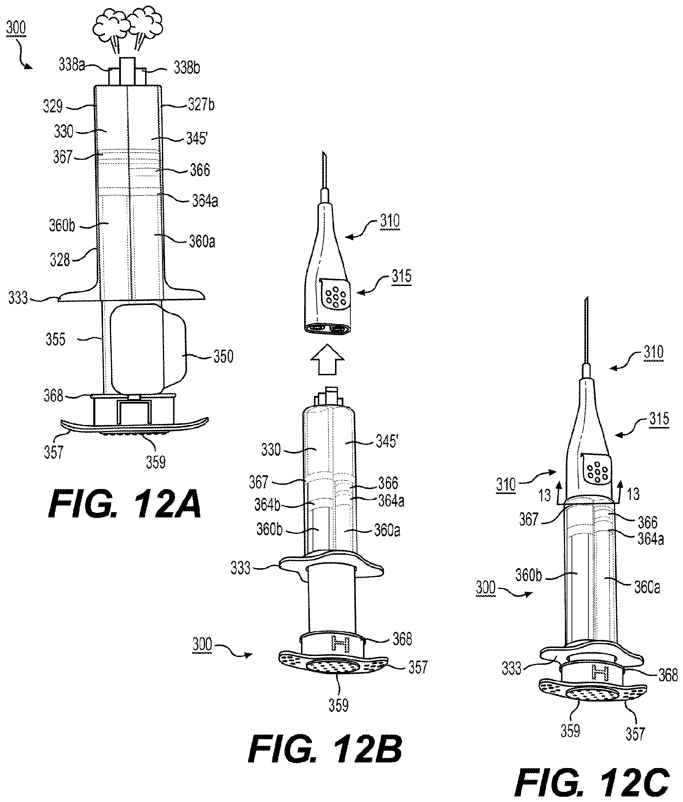

In Fig. 12A, cap 323 has been removed revealing ports 338a, 338b with precursor 345' formed and constituent 330 in chamber 329. Flange 357 and/or flange 359 can be distally advanced slightly to purge any air A from system 300 out through respective ports 338a, 338b. Retainer 350 can be positioned with respect to cavity 355 so as to contact flange 333 and prevent unwanted or otherwise unintended delivery of constituents from cavity 328 out through ports 338a, 338b. Preferably, air A is purged as shown while system 300 is oriented generally upward.

In Fig. 12B, system 300 is now being connected to connector 315, which has been primed and connected to needle 308 at the treatment site. In Fig. 12C, system 300 is now connected to connector 315. With system 300 ready and connector 315 primed, a user in Fig. 12C can advance flange 357 distally so that corresponding cavity 355 and rods 360a, 360b distally drive respective stoppers 364a, 366, 367, 369, and advance precursor 345' from chamber 327b and constituent 330 from chamber 329, through ports 338a, 338b, and into connector 315.

Fig. 13 is a partial cross-section view of section 13-13 of Fig. 12C showing example connector 315 assembled with a distal end of cavity 328. Chambers 327b, 329 can be in fluid communication with a proximal end of the central lumen 317 of connector 315, including via respective ports 338b, 338a. Lumen 317 can include a static mixer 353 so that constituent from respective chambers 327b, 329 can mix together and form the mixture to be delivered through needle 308.

In some aspects, connector 315 can include tube 358 with a proximal end configured in fluid communication with chamber 327b and pierce a corresponding membrane or seal of port 338b. Connector 315 can also include tube 362 with a proximal end configured in fluid communication with chamber 329 and pierce a corresponding membrane or seal of port 338a. In this respect, once precursor 345' is in position in chamber 327b and constituent 330 is positioned in chamber 329 and connector 315 assembled thereto, distally moving cavity 355 and/or rods 360a, 360b can cause precursor 345' and constituent 330 to egress through respective ports 338a, 338b and respective tubes 358, 362 to mix with each other in lumen 317. Tubes 358, 362 can form a Y-shape, as in Fig. 13, though any other shape can be used as needed or required.

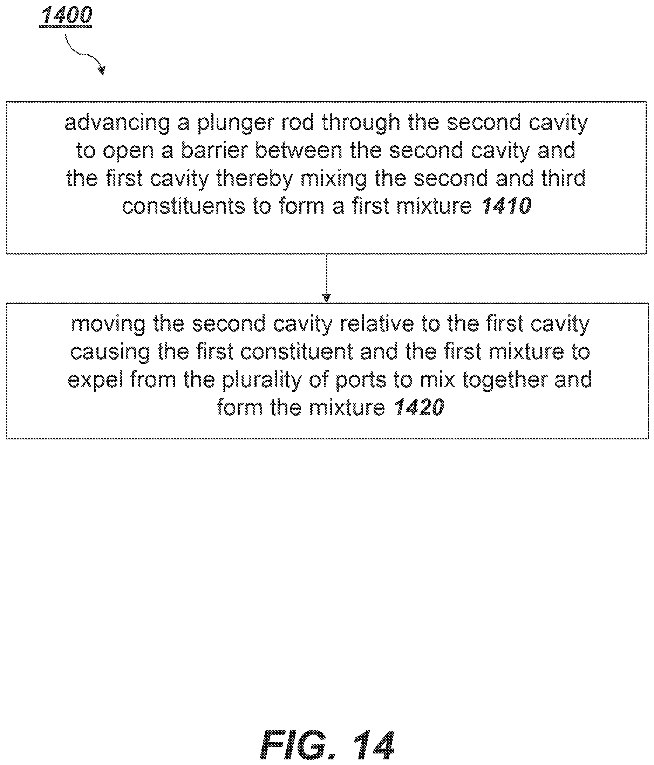

Fig. 14 depicts a method 1400 of using any of the herein disclosed mixing systems. Step 1410 of method 1400 can include advancing a plunger rod through the second cavity to open a barrier between the second cavity and the first cavity thereby mixing the second and third constituents to form a first mixture. Step 1420 of method 1400 can include moving the second cavity relative to the first cavity causing the first constituent and the first mixture to expel from the plurality of ports to mix together and form the mixture (e.g., filler 30 such as a gel composition). Method 1400 can end after step 1420. In other embodiments, additional steps according to the examples described above can be performed.

The systems and methods of this disclosure are beneficial by reducing the number of system components, are relatively simply to assemble and operate, with minimal mixing errors prior to delivery within a patient at a treatment site. Other aspects and embodiments of the present disclosure will be apparent to those skilled in the art from consideration of the specification and practice of the embodiments disclosed herein.

While certain features of the present disclosure are discussed within the context of exemplary procedures, the compositions, systems, and methods may be used for other medical procedures according to the general principles disclosed. The presently disclosed embodiments, therefore, are considered in all respects to be illustrative and not restrictive. It will therefore be apparent from the foregoing that while particular forms of the disclosure have been illustrated and described, various modifications of the invention, which fall under the scope of the claims, may be made. It is intended that the specification and examples be considered as exemplary only, with the scope of the invention being defined by the appended claims.

## Claims

1. A system (100) for producing a mixture to deliver to a treatment site, comprising:
a first cavity (128) comprising a distal end comprising a plurality of ports;
a second cavity (155) concentric with and advanceable through the first cavity (128), the second cavity (155) comprising:
a plunger on a distal end of the second cavity (155), the plunger being configured to move a first constituent (130) from the first cavity (128) through at least one of the plurality of ports, the first constituent (130) being distal of the plunger and comprised in an outer chamber (129) of the first cavity (128);
a plunger rod (160) advanceable through the second cavity (155) and configured to move a second constituent (140) from the first cavity (128) through at least one of the plurality of ports and mix together to form the mixture for delivery to the treatment site, the second constituent (140) being distal of the second cavity (155) and comprised in a central chamber (127b) of the first cavity (128),
**characterized in that** in a first state, moving the plunger rod (160) causes a third constituent (145) of the second cavity (155) to be delivered through a barrier to mix with the second constituent (140) to form a first mixture;
wherein in a second state, moving the plunger rod (160) and/or the second cavity (155) causes the first mixture and the first constituent (130) to be advanced through respective ports (138a, 138b).

2. The system (100) of Claim 1, wherein the outer chamber (129) is concentric with the central chamber (127b) comprising the second constituent (140).

3. The system (100) of Claim 2, further comprising a second outer chamber (127a) concentric with the central chamber (127b) comprising the second constituent (140).

4. The system (100) of Claim 1, wherein the outer chamber (129) runs parallel with the central chamber (127b) comprising the second constituent (140).

5. The system (100; 300) of Claim 4, wherein the plunger rod (160; 360) is divided into a pair of parallel plunger rods (360a, 360b), each of the parallel plunger rods (360a, 360b) advanceable within a respective chamber (327a, 327b) and by a common flange (359) on a proximal end of the plunger rods (360a, 360b).

6. The system (100; 300) of Claim 5, wherein the pair of plunger rods (360a, 360b) is configured to control the plunger.

7. The system (100) of any preceding claim, wherein the plunger is positioned between an outer surface of the second cavity (155) and an inner surface of the first cavity (128).

8. The system (100) of any preceding claim, wherein the third constituent (145) of the second cavity (155) is mixable with the second constituent (140) to form a precursor solution.

9. The system (100) of Claim 8, wherein the second and third constituents (140, 145) are separated by the barrier; and
wherein moving the plunger rod (160) and/or the second cavity (155) with respect to the first cavity (128) causes the barrier to open so the third constituent (145) bypasses the barrier and mixes with the second constituent (140) to form the precursor solution.

10. The system (100) of Claim 8, wherein the second and third constituents (140, 145) are separated by the barrier; and
wherein moving the plunger rod (160) and/or the second cavity (155) with respect to the first cavity (128) causes the barrier to toggle to an open state so the third constituent (145) mixes with the second constituent (140) to form the precursor solution.

11. The system (100; 200) of Claim 10, wherein the barrier is a floating plunger (166; 266) and the distal end of the second cavity (155; 255) comprises an internally positioned rib (269a);
wherein as the plunger rod (160; 260) is advanced distally, the floating plunger (166; 266) advances distally and contacts the rib (269a) to develop a moment causing the floating plunger (166; 266) to tilt and break a seal so constituent within the second cavity (155) can advance distal of the floating plunger (166; 266).

12. The system (100) of any preceding claim, wherein the plunger is a gasket or a septum.

## Patentansprüche

1. System (100) zur Herstellung eines Gemischs zur Abgabe an eine Behandlungstelle, umfassend:
einen ersten Hohlraum (128), der ein distales Ende umfasst, das eine Mehrzahl von Öffnungen umfasst;
einen zweiten Hohlraum (155), der konzentrisch zu dem ersten Hohlraum (128) ist und durch diesen hindurch vorbewegt werden kann, wobei der zweite Hohlraum (155) umfasst:
einen Kolben an einem distalen Ende des zweiten Hohlraums (155), wobei der Kolben dazu ausgestaltet ist, eine erste Komponente (130) aus dem ersten Hohlraum (128) durch mindestens eine der Mehrzahl von Öffnungen zu bewegen, wobei die erste Komponente (130) distal vom Kolben angeordnet ist und in einer äußeren Kammer (129) des ersten Hohlraums (128) enthalten ist;
eine Kolbenstange (160), die durch den zweiten Hohlraum (155) vorbewegt werden kann und dazu ausgestaltet ist, eine zweite Komponente (140) aus dem ersten Hohlraum (128) durch mindestens eine der Mehrzahl von Öffnungen zu bewegen und zusammen zu mischen zur Bildung des Gemischs zur Abgabe an die Behandlungsstelle, wobei die zweite Komponente (140) distal von dem zweiten Hohlraum (155) angeordnet ist und in einer zentralen Kammer (127b) des ersten Hohlraums (128) enthalten ist,
**dadurch gekennzeichnet, dass**
in einem ersten Zustand das Bewegen der Kolbenstange (160) bewirkt, dass eine dritte Komponente (145) des zweiten Hohlraums (155) durch eine Barriere hindurch abgegeben wird, um sich mit der zweiten Komponente (140) zu vermischen und ein erstes Gemisch zu bilden;
wobei in einem zweiten Zustand das Bewegen der Kolbenstange (160) und/oder des zweiten Hohlraums (155) bewirkt, dass das erste Gemisch und die erste Komponente (130) durch entsprechende Öffnungen (138a, 138b) vorbewegt werden.

2. System (100) nach Anspruch 1, wobei die äußere Kammer (129) konzentrisch zu der zentralen Kammer (127b) ist, die die zweite Komponente (140) enthält.

3. System (100) nach Anspruch 2, das ferner eine zweite äußere Kammer (127a) umfasst, die konzentrisch zu der zentralen Kammer (127b) ist, die die zweite Komponente (140) enthält.

4. System (100) nach Anspruch 1, wobei die äußere Kammer (129) parallel zu der zentralen Kammer (127b) verläuft, die die zweite Komponente (140) enthält.

5. System (100; 300) nach Anspruch 4, wobei die Kolbenstange (160; 360) in ein Paar paralleler Kolbenstangen (360a, 360b) unterteilt ist, wobei jede der parallelen Kolbenstangen (360a, 360b) innerhalb einer entsprechenden Kammer (327a, 327b) und durch einen gemeinsamen Flansch (359) an einem proximalen Ende der Kolbenstangen (360a, 360b) vorbewegt werden kann.

6. System (100; 300) nach Anspruch 5, wobei das Paar Kolbenstangen (360a, 360b) dazu ausgestaltet ist, den Kolben zu steuern.

7. System (100) nach einem der vorangehenden Ansprüche, wobei der Kolben zwischen einer Außenfläche des zweiten Hohlraums (155) und einer Innenfläche des ersten Hohlraums (128) angeordnet ist.

8. System (100) nach einem der vorangehenden Ansprüche, wobei die dritte Komponente (145) des zweiten Hohlraums (155) mit der zweiten Komponente (140) mischbar ist, um eine Vorläuferlösung zu bilden.

9. System (100) nach Anspruch 8, wobei die zweite und dritte Komponente (140, 145) durch die Barriere voneinander getrennt sind; und
wobei das Bewegen der Kolbenstange (160) und/oder des zweiten Hohlraums (155) relativ zum ersten Hohlraum (128) bewirkt, dass sich die Barriere öffnet, sodass die dritte Komponente (145) die Barriere passiert und sich mit der zweiten Komponente (140) vermischt, um die Vorläuferlösung zu bilden.

10. System (100) nach Anspruch 8, wobei die zweite und dritte Komponente (140, 145) durch die Barriere voneinander getrennt sind; und
wobei das Bewegen der Kolbenstange (160) und/oder des zweiten Hohlraums (155) relativ zum ersten Hohlraum (128) bewirkt, dass die Barriere zu einem offenen Zustand wechselt, sodass sich die dritte Komponente (145) mit der zweiten Komponente (140) vermischt, um die Vorläuferlösung zu bilden.

11. System (100; 200) nach Anspruch 10, wobei die Barriere ein schwimmender Kolben (166; 266) ist und das distale Ende des zweiten Hohlraums (155; 255) eine innen angeordnete Rippe (269a) umfasst;
wobei, wenn die Kolbenstange (160; 260) distal vorbewegt wird, der schwimmende Kolben (166; 266) distal vorbewegt wird und mit der Rippe (269a) in Kontakt kommt, um ein Moment zu erzeugen, das bewirkt, dass sich der schwimmende Kolben (166; 266) neigt und eine Versiegelung bricht, so dass Komponenten innerhalb des zweiten Hohlraums (155) distal des schwimmenden Kolbens (166; 266) vorbewegt werden können.

12. System (100) nach einem der vorangehenden Ansprüche, wobei der Kolben eine Dichtung oder ein Septum ist.

## Revendications

1. Système (100) pour produire un mélange destiné à être délivré sur un site de traitement, comprenant :
une première cavité (128) qui comprend une extrémité distale qui comprend une pluralité d'orifices ; et
une seconde cavité (155) qui est concentrique à la première cavité (128) et qui peut être avancée au travers de celle-ci, la seconde cavité (155) comprenant :
un plongeur sur une extrémité distale de la seconde cavité (155), le plongeur étant configuré pour déplacer un premier constituant (130) depuis la première cavité (128) au travers d'au moins l'un de la pluralité d'orifices, le premier constituant (130) étant distal par rapport au plongeur et étant compris dans une chambre externe (129) de la première cavité (128) ; et
une tige de plongeur (160) qui peut être avancée au travers de la seconde cavité (155) et qui est configurée pour déplacer un deuxième constituant (140) depuis la première cavité (128) au travers d'au moins l'un de la pluralité d'orifices et pour mélanger ensemble afin de former le mélange pour sa délivrance sur le site de traitement, le deuxième constituant (140) étant distal par rapport à la seconde cavité (155) et étant compris dans une chambre centrale (127b) de la première cavité (128),
**caractérisé en ce que**:
dans un premier état, le déplacement de la tige de plongeur (160) a pour effet qu'un troisième constituant (145) de la seconde cavité (155) est délivré au travers d'une barrière pour son mélange avec le deuxième constituant (140) afin de former un premier mélange ;
dans lequel, dans un second état, le déplacement de la tige de plongeur (160) et/ou de la seconde cavité (155) a pour effet que le premier mélange et le premier constituant (130) sont avancés au travers d'orifices respectifs (138a, 138b).

2. Système (100) selon la revendication 1, dans lequel la chambre externe (129) est concentrique à la chambre centrale (127b) qui comprend le deuxième constituant (140).

3. Système (100) selon la revendication 2, comprenant en outre une seconde chambre externe (127a) qui est concentrique à la chambre centrale (127b) qui comprend le deuxième constituant (140).

4. Système (100) selon la revendication 1, dans lequel la chambre externe (129) est développée parallèlement à la chambre centrale (127b) qui comprend le deuxième constituant (140).

5. Système (100 ; 300) selon la revendication 4, dans lequel la tige de plongeur (160 ; 360) est divisée selon une paire de tiges de plongeur parallèles (360a, 360b), chacune des tiges de plongeur parallèles (360a, 360b) pouvant être avancée à l'intérieur d'une chambre respective (327a, 327b) et au moyen d'une bride commune (359) sur une extrémité proximale des tiges de plongeur (360a, 360b).

6. Système (100 ; 300) selon la revendication 5, dans lequel la paire de tiges de plongeur (360a, 360b) est configurée pour commander le plongeur.

7. Système (100) selon l'une quelconque des revendications précédentes, dans lequel le plongeur est positionné entre une surface externe de la seconde cavité (155) et une surface interne de la première cavité (128).

8. Système (100) selon l'une quelconque des revendications précédentes, dans lequel le troisième constituant (145) de la seconde cavité (155) peut être mélangé avec le deuxième constituant (140) de manière à former une solution de précurseur.

9. Système (100) selon la revendication 8, dans lequel les deuxième et troisième constituants (140, 145) sont séparés par la barrière ; et dans lequel :
le déplacement de la tige de plongeur (160) et/ou de la seconde cavité (155) par rapport à la première cavité (128) a pour effet que la barrière est ouverte de telle sorte que le troisième constituant (145) passe outre la barrière et est mélangé avec le deuxième constituant (140) de manière à former la solution de précurseur.

10. Système (100) selon la revendication 8, dans lequel les deuxième et troisième constituants (140, 145) sont séparés par la barrière ; et dans lequel :
le déplacement de la tige de plongeur (160) et/ou de la seconde cavité (155) par rapport à la première cavité (128) a pour effet que la barrière passe dans un état ouvert de sorte que le troisième constituant (145) est mélangé avec le deuxième constituant (140) de manière à former la solution de précurseur.

11. Système (100 ; 200) selon la revendication 10, dans lequel la barrière est un plongeur flottant (166 ; 266) et l'extrémité distale de la seconde cavité (155 ; 255) comprend une nervure positionnée de façon interne (269a) ; et dans lequel :
lorsque la tige de plongeur (160 ; 260) est avancée de façon distale, le plongeur flottant (166 ; 266) est avancé de façon distale et entre en contact avec la nervure (269a), d'où le développement d'un moment qui a pour effet que le plongeur flottant (166 ; 266) est incliné et rompt une étanchéité de sorte qu'un constituant à l'intérieur de la seconde cavité (155) peut avancer de façon distale par rapport au plongeur flottant (166 ; 266).

12. Système (100) selon l'une quelconque des revendications précédentes, dans lequel le plongeur est un composant d'étanchéité ou un septum.
